# EUROPEAN PATENT APPLICATION

(11) **EP 2 706 662 A2**
(43) Date of publication of application: **12.03.2014**
(21) Application number: 13183348.5
(22) Date of filing: 06.09.2013
(51) Int. Cl.: H03G 1/02, H03G 3/30

(54) **System and method for remotely controlling audio equipment**

(30) Priority: 06.09.2012 US 201261697717 P
(71) Applicant: Music Group IP, Ltd., Road Town, Tortola (VG)
(72) Inventor: Behringer, Ulrich, 1227 MAKATI CITY, METRO MANILA (PH)
(74) Representative: EP&C

(57) **Abstract**

An innovative new series of personal near-field active monitor speakers incorporating a digital wireless receiver and an industry-standard digital serial data interface that provides for wireless and wired digital audio streaming, apps for mobile devices or computers (smart phones, tablets, laptops etc.) to provide remote configuration of studio monitor modeling, i.e., emulations of popular near-field reference monitors; artist signature modeling, i.e., emulations of the favorite settings of well-known recording artists and music producers; and remote room equalization using the microphone integrated in a mobile or desktop personal computer, or an attached external microphone.

## Description

### BACKGROUND

### Technical Field

The present disclosure relates to an audio system and related method for controlling audio system equipment and, more particularly, to a speaker or monitor system with integrated wireless communication and associated mobile device, such as a computer, tablet or smart phone, with application software to permit remote wireless configuration and digital audio streaming. Wired remote configuration and digital audio streaming from all types of mobile computer or desktop personal computer is also supported using an industry-standard digital serial data interface. Wireless remote configuration and digital audio streaming may also be performed from a desktop personal computer.

### Description of the Related Art

Public Address speakers, studio monitors and hi-fi speakers, to name a few, usually have fixed sound parameters (such as, but not limited to, crossover frequency, equalization, delays, limiter, etc.) and some form of variable controls (bass, treble, cut-off, volume controls, etc.) on the speaker itself to change the sound. Although nearly all audio equipment allows for easy adjustment of the variable controls, the fixed parameters are often difficult to alter on speakers, monitors and other equipment. In addition, some known audio systems employ a 30-pin or Ethernet, and the like, hardwired connection between the speakers and a controlling system to allow for some form of remote controllability, but this is limited by the need for a physical connection at all times, restricting the location from which a user can control the speakers.

Remotely controlling speakers and audio equipment has been done for many years. However, remote controls for audio equipment are generally only capable of controlling the variable parameters such as bass, treble and volume. Although this can be useful, it does not allow for full customization of the audio equipment because it requires many other parameters to remain fixed. In recent years the use of smart phone and tablet devices has become nearly ubiquitous, and remote controls for audio equipment can often now be implemented as applications to be loaded onto a smart phone or similar device. However, these remote control applications still suffer from the same deficiencies of standard remote controls, namely that they have only the ability to control the variable parameters such as volume, bass and treble and not the fixed parameters.

There is a need for audio systems that employ modern wireless remote control via a mobile computer devices, such as, but not limited to: iPad, iPhone, iPod touch, other iOS devices, Windows, Android, Chrome or Tizen devices or any other smart phone, tablet, laptop, notebook, netbook or mobile device, referred to generally as a mobile computer, where parameters of the speakers can be controlled through Bluetooth or Wi-Fi, and the like, that facilitate changing the parameters of the speaker beyond merely volume, treble and bass. Wired remote configuration and digital audio streaming from all types of mobile computer or desktop personal computer is also supported using an industry-standard digital serial data interface. Wireless remote configuration and digital audio streaming may also be performed from a desktop personal computer.

### BRIEF SUMMARY

In accordance with a representative embodiment of the present disclosure, an audio system is provided that includes a mobile computer having a user interface configured to receive user input, a memory coupled to the user interface and having a sequence of instructions stored thereon that, when executed by a processor in the mobile control system, generate control signals in response to user input received at the user interface, and a wireless transmitter configured to transmit the control signals and streamed digital audio. The mobile computer interacts with a speaker system having at least one active speaker, a wireless receiver for wireless stereo audio streaming and remote control, at least one analog input terminal configured to receive analog signals, at least one digital serial data interface for wired stereo audio streaming and remote control, and a digital signal processor configured to process the control signals received by the wireless receiver and any received audio signals and control operation of the at least one speaker in response to the received control signals and any received audio signals.

In accordance with another aspect of the present disclosure, the speaker system may include at least one passive speaker coupled to the active speaker, or it may include or substitute at least one passive speaker coupled to external amplification, digital signal processing, and a digital wireless receiver.

In a preferred system, the digital signal processor is enabled to configure a response of the at least one active speaker.

In accordance with still yet a further aspect of the present disclosure, the configuration of a response includes at least one from among models of other speaker settings, settings of well-known music artists, and settings of music producers.

In accordance with yet another aspect of the present disclosure, the mobile computer utilizes at least one audio transducer configured to convert sound into electronic signals, such as the microphone integrated in the mobile computer, or an attached external microphone in some embodiments, and is used in conjunction with the digital signal processor of the speaker system that is configured to perform equalization of audio output of the at least one active speaker (or at least one passive speaker in combination with external amplification, digital signal processing and digital wireless receiver) in response to audio sounds received at the at least one audio transducer.

In accordance with another aspect of the present disclosure, a system is provided that includes a mobile computer having a memory and a wireless transmitter, the memory storing a software program, and the wireless signal transmitter configured to transmit a controlling signal to an audio equipment based on a user instruction; a user interface coupled to the mobile computer and configured to receive an input; a digital wireless receiver coupled to the audio equipment and configured to receive the controlling signal and streamed digital audio; and a controller coupled to the digital wireless receiver and configured to alter a sound parameter of the audio equipment in response to the controlling signal. Wired remote configuration and digital audio streaming from all types of mobile computer or desktop personal computer is also supported using an industry-standard digital serial data interface. Wireless remote configuration and digital audio streaming may also be performed from a desktop personal computer.

As will be readily appreciated from the foregoing and the accompanying detailed description, an innovative new series of personal near-field active monitor speakers incorporating a digital wireless receiver is provided in accordance with a representative embodiment of the present disclosure.

### BRIEF DESCRIPTION OF THE SEVERAL VIEWS OF THE DRAWINGS

The foregoing and other features and advantages of the present disclosure will be more readily appreciated as the same become better understood from the following detailed description when taken in conjunction with the accompanying drawings, wherein:
Figure 1 is an illustration of the topology of one embodiment of an audio system formed in accordance with the present disclosure with one two-way active speaker;
Figure 2 is a block diagram of one preferred embodiment of a speaker system employing the design of the present disclosure with one two-way active speaker; and
Figure 3 is a flowchart of an automatic equalization of a speaker system according to one embodiment of the present disclosure.

### DETAILED DESCRIPTION

In the following description, certain specific details are set forth in order to provide a thorough understanding of various disclosed embodiments. However, one skilled in the relevant art will recognize that embodiments may be practiced without one or more of these specific details, or with other methods, components, materials, etc. In other instances, well-known structures or components or both associated with monitors, speakers, personal mobile electronic devices such as, but not limited to, iPad, iPhone, iPod touch, other iOS device, Windows, Android, Chrome or Tizen device or any other smart phone, tablet, laptop, notebook, netbook or mobile device, referred to herineafter generally as a mobile computer, as well as, personal computers, loudspeakers, and the like have not been shown or described in order to avoid unnecessarily obscuring descriptions of the embodiments.

Unless the context requires otherwise, throughout the specification and claims that follow, the word "comprise" and variations thereof, such as "comprises" and "comprising" are to be construed in an open inclusive sense, that is, as "including, but not limited to." The foregoing applies equally to the words "including" and "having."

Reference throughout this description to "one embodiment" or "an embodiment" means that a particular feature, structure, or characteristic described in connection with the embodiment is included in at least one embodiment. Thus, the appearance of the phrases "in one embodiment" or "in an embodiment" in various places throughout the specification are not necessarily all referring to the same embodiment. Furthermore, the particular features, structures, or characteristics may be combined in any suitable manner in one or more embodiments.

The representative embodiments of the present disclosure will be described in the context of near-field monitors. It is to be understood, however, that the concepts and disclosures herein can be applied to speakers in general, such as loud speakers, stereo speakers, home audio speakers, and the like.

Generally, in the present disclosure, a wireless remote control is implemented with a mobile device, referred to herineafter as a mobile computer. The mobile computer can include, but is not limited to, iPad, iPhone, iPod touch, other iOS devices, Windows, Android, Chrome or Tizen device or any other smart phone, tablet, laptop, notebook, netbook or mobile device, that is configured to control essentially all parameters of the speakers through Bluetooth, Wi-Fi or other wireless technology. This is accomplished by changing the parameters of the speaker via an analog crossover with controllable analog switches or, more preferably, with a digital signal processor (DSP) that is connected to a Bluetooth, Wi-Fi or other wireless technology receiver. Wired remote configuration and digital audio streaming from all types of mobile computer or desktop personal computer is also supported using an industry-standard digital serial data interface. Wireless remote configuration and digital audio streaming may also be performed from a desktop personal computer.

This remote controllability of the speaker parameters allows for (1) control of various sound presets offered by the provider; (2) "Sound modeling" of well-established competitor speakers; and (3) "Signature Series" where presets by famous sound engineers or artists are provided (for example a Quincy Jones Preset that allows a user to listen to sound produced from the speakers using settings that Quincy Jones uses for configuring his personal or professional speakers).

Ideally, the foregoing is implemented through applications that the user can download and use to control the sound of the speakers with the mobile computer described above. This allows a user to alter the parameters of the audio equipment from a remote location, such as a chair within the audience or in a recording studio. The application can also show graphic displays of the speakers they are modeled after or the artists that provided the presets.

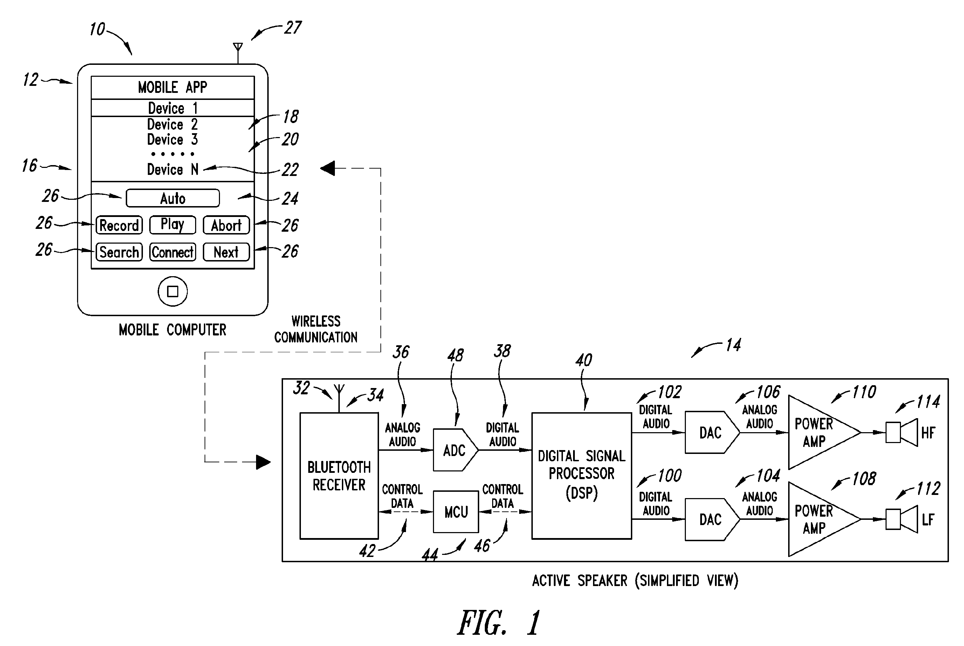

Referring to Figure 1, shown therein is an audio system 10 having a mobile computer 12 and a speaker or near field monitor system 14. A mobile control system, generally a mobile computer, is shown in the form of a personal communication device, such as a smart phone 16 having a user interface 18 in the form of a touch screen 20 displaying a selection of devices 22 to be controlled along with a control panel 24 having buttons 26. The mobile computer is a known processing device that is readily commercially available and will not be discussed in detail herein. Briefly, it includes a processor, internal memory, a display device that functions as the user interface 18, such as the touch screen 20, and is configured to process instructions stored in the memory in response to user input on the display device 18 or other known input device, such as a keyboard or mouse or any combination of the foregoing, such instructions provided in the form of a software application, commonly referred to as an "App".

The user interface 18 is configured to enable a user to select which device 22 to control, such as an active speaker, a passive speaker, or other audio equipment. The user also utilizes the user interface 18 to use the display screen and either soft or hard keys to select which functions to control, including changing parameters of the audio equipment and controlling streaming digital audio. Generally, the user will interact with the user interface 18 through a series of touch sensitive buttons 26 displayed on the touch screen 20. For example, the user interface may display buttons with labels such as: audio, record, play, abort, search, connect, and next.

The mobile computer 12 also has wireless communication capability, including a wireless transmitter with antenna 27 to transmit control signals, such as digital wireless signals, and stream digital audio. The wireless transmitter with antenna 27 is coupled to the mobile computer 12 and configured to transmit a radio frequency signal such as, but not limited to, Wi-Fi and Bluetooth from the mobile computer to the audio equipment. The wireless transmitter with antenna 27 may be natively integrated into the mobile computer, or an after-market transmitter coupled to the mobile computer may be used.

The speaker system 14 shown in Figure 1 includes an antenna 32 configured to receive digital wireless signals, and a digital wireless receiver module 34. The analog audio output 36 of digital wireless receiver module 34 is coupled to an analog-to-digital converter (ADC) 48, the digital output 38 of which is then in turn coupled to the input of Digital Signal Processor (DSP) 40. Control of both the digital wireless receiver 34 and Digital Signal Processor 40 is achieved using microcontroller (MCU) 44 which communicates via serial control data links 42 and 46. The two digital audio outputs 100 and 102 from the DSP feed digital-to-analog converters (DAC) 104 and 106 in order to convert the processor's signals to analog. The analog signals are then fed to power amplifiers 108 and 110 which drive Low Frequency and High Frequency speakers 112 and 114.

The antenna 32 and wireless receiver module 34 receive transmitted signals from the wireless transmitter with antenna 27. The antenna 32 and wireless receiver module 34 are configured to receive radio frequency signals such as, but not limited to, Wi-Fi or Bluetooth. The received signals may be control signals that are received by the microcontroller (MCU) 44 which in turn instructs the DSP 40 to configure the audio parameters of the audio equipment or to alter a parameter of the audio equipment, or they may be digital audio signals that are received and processed by DSP 40 and played through the speaker or other audio equipment.

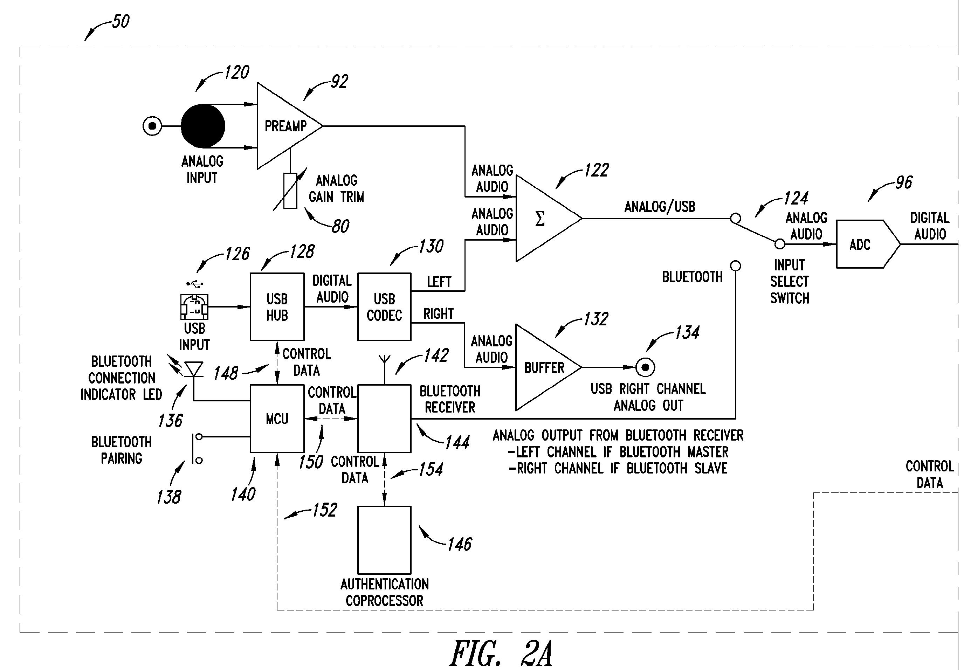

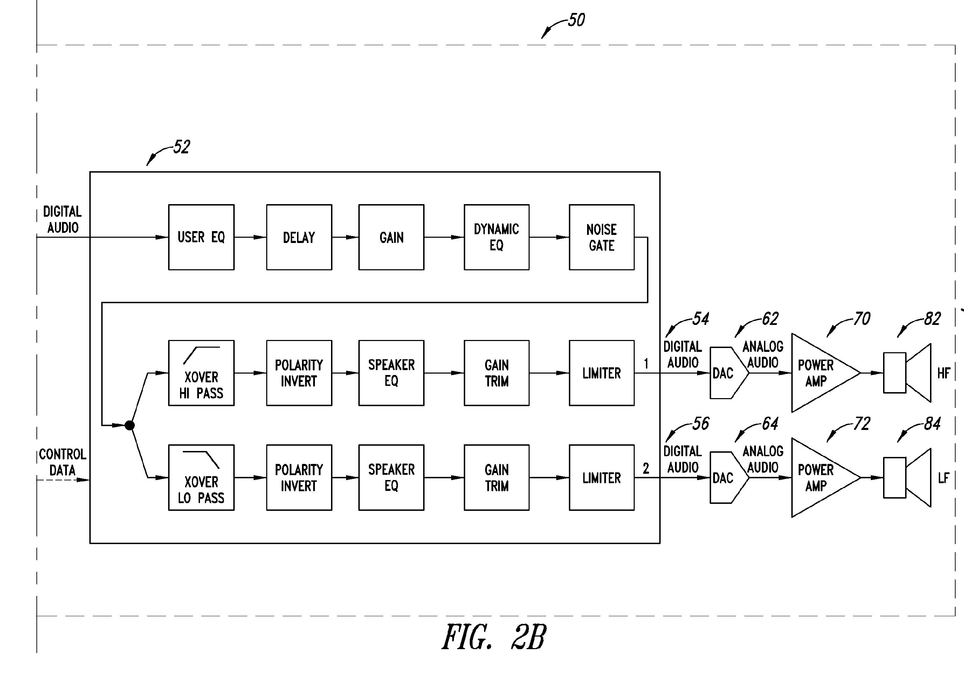

Figure 2 illustrates one preferred embodiment of the present disclosure in connection with an active speaker system 50. In the standard configuration, a 1-input, 2-output DSP 52 which implements a two-way crossover with separate low-pass and high-pass filters (XOVER LO PASS and XOVER HI PASS respectively) is used, is configured as shown in Figure 2. The DSP 52 is configured to perform pre-crossover functions such as, but not limited to, user equalization (USER EQ), delay, gain, dynamic equalization (DYNAMIC EQ) and noise gate functions for the input channel, and post-crossover functions such as, but not limited to, polarity invert, speaker equalization (SPEAKER EQ), gain trim and limiter for each of the two output channels 54, 56. The pre-crossover functions are generally intended for user adjustment from the mobile computer application, whereas the crossover and post-crossover functions are specific to the speakers used and are not generally intended for user adjustment, however access may be permitted using an "expert user" or diagnostic mode in the mobile computer application. In one preferred embodiment, external digital-to-analog converters (DAC) 62, 64 will be required to convert the digital output back to analog to feed the power amplifiers 70, 72 which in turn drive high frequency (HF) speaker 82 and low frequency (LF) speaker 84 respectively.

The active speaker system 50 also includes a wireless Bluetooth receiver 144 with antenna 142 configured to receive control signals from a wireless transmitter, such as the wireless transmitter with antenna 27 of the mobile computer of Figure 1.

It is recognized that in a stereo configuration of two such identical active speakers, that the Mobile Computer may first need to be wirelessly paired to one of the active speakers (which becomes the Bluetooth Master speaker) which in turn may then be paired with the other speaker (the Bluetooth Slave speaker). As the two speakers are identical, to ensure that the left and right audio channels are heard from the left- and right-positioned speakers respectively, a simple rule can be made that the user should always pair the mobile computer with, say, the left active speaker first, such that this then becomes the Bluetooth Master speaker. The left active speaker is then paired with the right active speaker which becomes the Bluetooth Slave speaker (or vice versa if the speaker is so designed that the right channel speaker should always be the Bluetooth Master). The Bluetooth Receiver module 144 is correspondingly programmed to output the left audio channel on its analog output if it is set as the Bluetooth Master or to output the right audio channel if it is the Bluetooth Slave. Pairing between the Mobile Computer and the Bluetooth Master left speaker, and between the Bluetooth Master left speaker and the Bluetooth Slave right speaker is performed using Bluetooth Pairing switch 138, with Pairing Connection status indicated by light emitting diode (LED) 136.

In one preferred embodiment, the active speaker will need to be certified under the Apple MFi program as the Bluetooth communication will use Apple protocols and an Apple Authentication Coprocessor 146 will be required to be connected to the Bluetooth Receiver Module 144 via serial control data connection 154.

In one preferred embodiment, balanced and unbalanced analog inputs are provided using industry-standard audio connectors 120, and a preamplifier 92 with an externally adjustable gain trim control 80 is provided for setting the optimum input signal level.

A standard wired computer serial data interface, such as, but not limited to, Universal Serial Bus (USB) is provided in one embodiment for stereo audio streaming and remote control, as an alternative to wireless audio streaming and control. In one preferred embodiment, when a stereo pair of speakers is used, the serial data interface of the left channel speaker will be used to connect to the transmitting device, however, in other embodiments the right speaker could be used. The USB interface 126 is connected to a USB hub 128 under the control of microcontroller (MCU) 140 and after conversion to stereo analog audio signals by a USB Codec 130, the left channel of this stereo audio signal is summed with the analog input preamplifier output by summing amplifier 122, whilst the right channel is routed to an external unbalanced audio connector 134 via a buffer amplifier 132, for wired connection to the right channel speaker in a stereo configuration. In this way, the routing of the left and right channel audio signals corresponds with the relative physical placement of the speakers. The microcontroller (MCU) 140 interfaces to the USB hub 128, Bluetooth receiver 144 and DSP 52 via serial control data connections 148, 150 and 152 respectively.

An external two-position switch 124 is used to select between the analog output of summing amplifier 122 and the analog output of the Bluetooth Receiver module 144 (the left audio channel if the Bluetooth Receiver is Bluetooth Master and the right audio channel if it is Bluetooth Slave). The selected input connects to Analog-to-Digital Converter (ADC) 96 and the respective digital audio output signal is then received at respective input terminal of the DSP 52.

Configuration of the speaker settings in the DSP 52 may be performed over the wireless link to the speaker 50. Other speaker configurations (e.g., stereo, 5.1 surround sound, etc.) can also be created using combinations of speakers.

In one preferred embodiment, the wireless Bluetooth receiver 144 will mute its analog output when there is no valid connection established with a mobile computer to avoid undesired audible noises being heard.

Referring to Figure 1, another feature is the use of a software application program (commonly referred to as an "App") typically downloaded to the mobile computer by a user to configure the responses of speakers, such as speaker 14, to emulate other models of studio monitors and the favorite settings of well-known music artists and producers via the remote wireless connection. These emulations contain pre-set parameter settings for the DSP functions as shown in Figure 2. Referring again to Figure 1, the emulation settings may come pre-loaded onto the mobile computer application or may be downloaded or entered manually after the smartphone application has been loaded. The user will be able to select certain emulations by interacting with the user interface 18. Once a selection is made, the mobile computer 12 will transmit a control signal through the transmitter 36 to the audio equipment, which will automatically change the parameters of the speaker in response to the control signals and incorporate the parameters selected by the user. Once the DSP adjusts the parameters of the speaker 14 according to the selection made, the user will be able to listen to an audio output that will emulate other speaker models or the configurations that other artists or producers use.

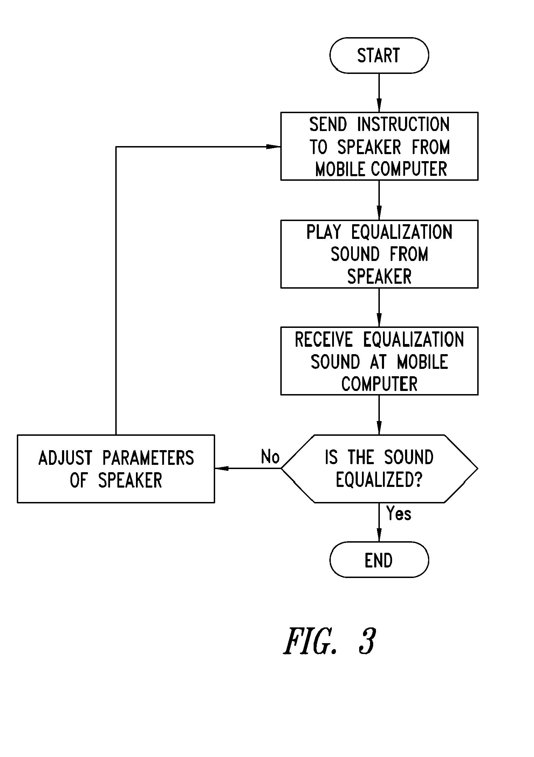

As shown in Figure 3, another feature of the present disclosure is the ability to provide for room equalization using the mobile computer 12. Room equalization will be performed using the application and a microphone integrated into the mobile computer or coupled to the mobile computer, in conjunction with the DSP integrated into an active speaker in one embodiment. The mobile computer 12 sends a signal to the speaker 14 to enter into a room equalization mode. The speaker 14 then outputs a test noise from the speaker, which the microphone coupled to the mobile computer 12 senses or receives. The mobile computer processes the received test noise from the speaker, comparing various parameters of the received test noise to ideal settings or preferred settings made by a user. As a result of the comparison, a comparison signal representing a change to be made in the speaker output parameters or settings, is generated by the mobile computer.

The mobile computer then sends the generated comparison signal back to the speaker (either wired or wirelessly), where it performs adjustments to the parameters of the speaker if necessary. The mobile computer and speaker may perform this testing and adjustment multiple times in order to fully equalize all parameters. The end result is an equalized sound for the listener's position without the need to move around to adjust parameters manually.

As an alternative to the use of a mobile computer, an integrated or external microphone coupled to a desktop personal computer may be used.

Embodiments of the invention may be described by one or more of the following clauses
1. An audio system, comprising:
   a mobile computer having:
      a user interface configured to receive user input;
      a memory coupled to the user interface and having a sequence of instructions stored thereon that when executed by a processor in the mobile computer generate control signals in response to user input received at the user interface;
      a transmitter configured to transmit the control signals;
   a speaker system having:
      at least one speaker;
      a wireless receiver configured to receive the control signals and to convert received streamed digital audio into an analog signal or signals;
      a microcontroller configured to receive data messages from the wireless receiver and generate corresponding messages;
      at least one analog input terminal configured to receive the analog signals;
      at least one industry-standard digital serial data interface configured to receive the control signals and streamed digital audio; and
      a digital signal processor configured to process the control signals received by the wireless receiver and configured to control operation of the at least one speaker in response to the received control signals through generation of analog signals in response to the control signals.
2. The audio system of clause 1, where a desktop personal computer is substituted for a mobile computer
3. The audio system of clause 1, wherein the speaker is an active speaker.
4. The audio system of clause 3 wherein the speaker system includes at least one passive speaker coupled to the active speaker.
5. The audio system of clause 1, wherein the speaker is a passive speaker configured to be coupled to an external amplification device, external digital signal processing, and an external digital wireless receiver.
6. The audio system of clause 1, wherein the mobile computer is configured to transmit digital audio signals to the speaker system to play through the speaker.
7. The audio system of clause 6, wherein the received digital audio signal is processed by the digital signal processor.
8. The audio system of clause 1, wherein the digital signal processor is structured to configure a parameter of the at least one speaker in response to the received control signals.
9. The audio system of clause 8, wherein the parameter configuration includes at least one from among settings of other speaker models, settings of well-known music artists, and settings of music producers.
10. The audio system of clause 1 wherein the mobile computer includes at least one audio transducer configured to convert sound into electronic signals, and the digital signal processor of the speaker system is configured to perform equalization of audio output of the at least one speaker in response to audio sounds received at the at least one audio transducer.
11. The audio system of clause 1, wherein controlling operation of the speaker comprises adjusting at least one of: user equalization, delay, gain, dynamic equalization, noise gate, polarity invert, speaker equalization, gain trim and limiter.
12. The audio system of clause 1, the speaker system further comprising:
   a first speaker;
   a second speaker identical to the first active speaker;
   wherein a selection of a left and right channel for each speaker corresponding to a relative physical placement of the speakers is determined by an order in which they are wirelessly paired with the mobile computer.
13. The audio system of clause 1, the speaker system further comprising:
   a active speaker having a first industry-standard digital serial data interface;
   a second speaker identical to the first speaker having a second industry-standard digital serial data interface;
   wherein a selection of a left and right channel for each speaker corresponding to a relative physical placement of the speakers is determined by the choice of which of the first and second industry-standard digital serial data interfaces is used to connect to the transmitting device.
14. A system, comprising:
   a mobile computer including a memory and a wireless transmitter, the memory configured to store a software program, and the wireless transmitter configured to transmit a controlling signal and a streamed digital audio signal to an audio equipment based on a user instruction;
   a user interface coupled to the mobile computer and configured to receive an input and to generate the user instruction;
   a receiver coupled to the audio equipment and configured to receive the controlling signal and streamed digital audio; and
   a controller coupled to the receiver and configured to alter a sound parameter of the audio equipment in response to the controlling signal.
15. The audio system of clause 14, where a desktop personal computer is substituted for a mobile computer
16. The system of clause 14, wherein the receiver is configured to receive at least one of Wi-Fi and Bluetooth signals.
17. The system of clause 14, wherein the controller comprises a microcontroller and/or a digital signal processor.
18. The system of clause 14, wherein the sound parameter of the audio equipment consist of at least one from among: user equalization, delay, gain, dynamic equalization, noise gate, polarity invert, speaker equalization, gain trim and limiter.
19. The system of clause 14, wherein altering the sound parameter includes configuring the parameter to at least one from among: settings of other speaker models, settings of well-known music artists, and settings of music producers.
20. A system, comprising:
   at least one electronic audio device having a wireless receiver, an industry-standard digital serial data interface and a controller,
   the wireless receiver configured to receive a wireless control signal and streamed digital audio and the controller configured to adjust an audio output in response to the received control signal;
   the industry-standard digital serial data interface configured to receive control signal and streamed digital audio via a wired cable connection and the controller configured to adjust an audio output in response to the received control signal;
   a mobile computer having a user interface, a memory, microphone, and a wireless transmitter, the mobile computer including a series of instructions stored in the memory and configured to carry out the following:
      receive input via the user interface;
      generate a control signal in response thereto and transmit the control signal via the wireless transmitter to the at least one electronic audio device; and
      sense through the microphone an audio sound from the electronic audio device and transmit a results signal to the at least one electronic audio device.
21. The system of clause 20, wherein the microphone is an external microphone configured to be coupled to the mobile computer.
22. The system of clause 20, wherein the wireless receiver is configured to receive a radio frequency signal.
23. The system of clause 20, wherein the industry-standard digital serial data interface is configured to receive data messages from a mobile or desktop personal computer via a wired cable connection.
24. The system of clause 20, wherein the at least one electronic audio device is a speaker.
25. The system of clause 20, wherein adjusting the audio output includes changing at least one parameter that includes one from among: user equalization, delay, gain, dynamic equalization, noise gate, polarity invert, speaker equalization, gain trim and limiter.
26. The system of clause 25, wherein the parameter is changed to at least one of: a setting of other speaker models, a setting of well-known artists, and a setting of music producers.
27. The system of clause 20, wherein the electronic audio equipment performs equalization of the audio output in response to the received results signal.

These and other features can be incorporated into the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

## Claims

1. An audio system, comprising:
a mobile computer having:
a user interface configured to receive user input;
a memory coupled to the user interface and having a sequence of instructions stored thereon that when executed by a processor in the mobile computer generate control signals in response to user input received at the user interface;
a transmitter configured to transmit the control signals;
a speaker system having:
at least one speaker;
a wireless receiver configured to receive the control signals and to convert received streamed digital audio into an analog signal or signals;
a microcontroller configured to receive data messages from the wireless receiver and generate corresponding messages;
at least one analog input terminal configured to receive the analog signals;
at least one industry-standard digital serial data interface configured to receive the control signals and streamed digital audio; and
a digital signal processor configured to process the control signals received by the wireless receiver and configured to control operation of the at least one speaker in response to the received control signals through generation of analog signals in response to the control signals.

2. The audio system of claim 1, wherein the speaker is an active speaker, wherein the speaker system preferably includes at least one passive speaker coupled to the active speaker.

3. The audio system of claim 1, wherein the speaker is a passive speaker configured to be coupled to an external amplification device, external digital signal processing, and an external digital wireless receiver.

4. The audio system of any of the claims 1-3, wherein the mobile computer is configured to transmit digital audio signals to the speaker system to play through the speaker,
wherein the received digital audio signal is preferably processed by the digital signal processor.

5. The audio system of any of the claims 1-4, wherein the digital signal processor is structured to configure a parameter of the at least one speaker in response to the received control signals,
wherein the parameter configuration preferably includes at least one from among settings of other speaker models, settings of well-known music artists, and settings of music producers.

6. The audio system of any of the claims 1-5, wherein the mobile computer includes at least one audio transducer configured to convert sound into electronic signals, and the digital signal processor of the speaker system is configured to perform equalization of audio output of the at least one speaker in response to audio sounds received at the at least one audio transducer.

7. The audio system of any of the claims 1-6, wherein controlling operation of the speaker comprises adjusting at least one of: user equalization, delay, gain, dynamic equalization, noise gate, polarity invert, speaker equalization, gain trim and limiter.

8. The audio system of any of the claims 1-7, the speaker system further comprising:
a first speaker;
a second speaker identical to the first active speaker;
wherein a selection of a left and right channel for each speaker corresponding to a relative physical placement of the speakers is determined by an order in which they are wirelessly paired with the mobile computer.

9. The audio system of any of the claims 1-8, the speaker system further comprising:
a active speaker having a first industry-standard digital serial data interface;
a second speaker identical to the first speaker having a second industry-standard digital serial data interface;
wherein a selection of a left and right channel for each speaker corresponding to a relative physical placement of the speakers is determined by the choice of which of the first and second industry-standard digital serial data interfaces is used to connect to the transmitting device.

10. A system, comprising:
a mobile computer including a memory and a wireless transmitter, the memory configured to store a software program, and the wireless transmitter configured to transmit a controlling signal and a streamed digital audio signal to an audio equipment based on a user instruction;
a user interface coupled to the mobile computer and configured to receive an input and to generate the user instruction;
a receiver coupled to the audio equipment and configured to receive the controlling signal and streamed digital audio; and
a controller, preferably comprising a microcontroller and/or a digital signal processor, coupled to the receiver and configured to alter a sound parameter of the audio equipment in response to the controlling signal.

11. The audio system of any of the preceding claims, where a desktop personal computer is substituted for a mobile computer.

12. The system of claim 10 or 11, wherein the receiver is configured to receive at least one of Wi-Fi and Bluetooth signals.

13. The system of claim 14, wherein the sound parameter of the audio equipment consist of at least one from among: user equalization, delay, gain, dynamic equalization, noise gate, polarity invert, speaker equalization, gain trim and limiter, and/or
wherein altering the sound parameter includes configuring the parameter to at least one from among: settings of other speaker models, settings of well-known music artists, and settings of music producers.

14. A system, comprising:
at least one electronic audio device, preferably a speaker, having a wireless receiver, an industry-standard digital serial data interface and a controller,
the wireless receiver configured to receive a wireless control signal and streamed digital audio and the controller configured to adjust an audio output in response to the received control signal;
the industry-standard digital serial data interface configured to receive control signal and streamed digital audio via a wired cable connection and the controller configured to adjust an audio output in response to the received control signal;
a mobile computer having a user interface, a memory, microphone, and a wireless transmitter, the mobile computer including a series of instructions stored in the memory and configured to carry out the following:
receive input via the user interface;
generate a control signal in response thereto and transmit the control signal via the wireless transmitter to the at least one electronic audio device; and
sense through the microphone an audio sound from the electronic audio device and transmit a results signal to the at least one electronic audio device.

15. The system of claim 14, comprising one or more of the following features,
- wherein the microphone is an external microphone configured to be coupled to the mobile computer;
- wherein the wireless receiver is configured to receive a radio frequency signal;
- wherein the industry-standard digital serial data interface is configured to receive data messages from a mobile or desktop personal computer via a wired cable connection;
- wherein adjusting the audio output includes changing at least one parameter that includes one from among: user equalization, delay, gain, dynamic equalization, noise gate, polarity invert, speaker equalization, gain trim and limiter;
- wherein the parameter is changed to at least one of: a setting of other speaker models, a setting of well-known artists, and a setting of music producers; and/or.
- wherein the electronic audio equipment performs equalization of the audio output in response to the received results signal.
